# EUROPEAN PATENT APPLICATION

(11) **EP 3 494 878 A1**
(43) Date of publication of application: **12.06.2019**
(21) Application number: 16908725.1
(22) Date of filing: 11.07.2016
(51) Int. Cl.: A61B 5/05

(54) **INTRAOPERATIVE MONITORING SYSTEM**

(71) Applicant: Jimenez-Carles Gil-Delgado, Eduardo, 28692 Villafranca del Castillo (Madrid) (ES)
(72) Inventor: Jimenez-Carles Gil-Delgado, Eduardo, 28692 Villafranca del Castillo (Madrid) (ES)
(74) Representative: Naranjo Marcos, Maria Antonia
(86) International application number: PCT/ES2016/070522
(87) International publication number: WO 2018/011439

(57) **Abstract**

The invention relates to an intraoperative monitoring system comprising a series of sensors (4) or stimulators placed on the body of a patient (1) and connected to one or more wireless signal-conditioning/-transmitting elements (ATX (6)) using small cables (3') and one or more wireless receiving/actuating elements (RXA (7)) connected to the monitoring system. The wireless signal-conditioning/-transmitting elements (ATX (6)) can be positioned beneath the operating table or stretcher.

## Description

### Field of the invention

The invention relates to a wireless intraoperative monitoring system that will minimize wiring and give surgeons more room during operations.

### Background

Intraoperative monitoring (IONM) consists of inserting a series of electrodes and stimulation probes into the patient, connected to amplifiers and/or stimulators to control the integrity of the patient's nervous system, i.e. to check the nervous links between the brain and the different parts of the body. These cables (1.5 or 2 m or more in length) are connected to a large device, creating physical barriers in the operating theatre and limiting spaces where other professionals need to work or move. The electrodes can also be accidentally disconnected, causing the records and monitoring to fail and/or the loss of critical information.

The monitoring techniques enable continuous assessment of the structures at risk. They assess the functioning of certain nervous system pathways while the surgical operation is taking place. The advantage is that they detect imminent damage to the monitored pathways, making it possible to act accordingly to avoid such damage.

Surgical intervention on the motor nerves leads to mechanical nervous irritations which, in turn and through action potentials, generate innervated musculature contractions. These contractions can be measured with needle or surface electrodes (EMG) and can be represented acoustically by a loudspeaker or optically on a screen. The surgeon 'hears' or sees any muscle activity caused during the operation. The nerve can be stimulated simultaneously with a special stimulation probe that leads to muscle contractions if it is still intact.

The purpose of IONM is to assist the surgical team in intraoperative decision-making, helping to reduce the incidence of permanent postoperative neurological damage.

IONM is a sub-speciality of neurophysiology. Although it is performed mostly by neurophysiologists, there are centres in which the task is assumed by anaesthesiologists or neurosurgeons. In any case, its complexity today requires the exclusive dedication of a specialist throughout the operation.

The absence of alterations in the monitored pathway does not imply the integrity of the remaining functional pathways of the central nervous system. Since there is an anatomical and functional division of the various pathways (sensory, motor, etc.), one of them may be damaged inadvertently if it is not monitored. No single form of monitoring covers all the functions of the nervous system. Therefore, IONM must be multimodal: all the functional modalities at risk during the operation must be monitored (and there are usually more than one). However, the presence of too many cables increases the risk of conflict with the surgeons' movements and prevents them from doing their work correctly.

### Description of the invention

The invention is a system of wireless recording and stimulation electrodes that will improve the functioning of the system in terms of monitoring, recording and stimulation as applied to patients undergoing surgery with intraoperative monitoring, as well as in epilepsy tests and deep brain stimulation surgery, simplifying the installation of the measuring, recording and stimulation devices and making it easier for surgeons to carry out the operation and neurophysiologists and neurologists to work with the system on patients.

The system that has been developed comprises one or more conditioning-transmitting elements (ATX) and, in general, one receiver-actuator element (RXA).

The ATX is composed of an ultra-low noise signal-conditioning system powered by a battery and designed according to safety criteria for biomedical measuring devices with internal electrodes, and a radio conversion, coding and transmission system. Optionally, it can also perform other functions such as measuring the contact impedance between the electrodes, acquiring non-electrical data and receiving configuration messages via the corresponding link. Another version will also be able to receive stimulation messages and commands and apply them to the patient.

The RXA contains a radio receiver and the electronics necessary to produce signals at the outputs that are an exact or amplified copy of the input signals to the ATX. Optionally, it can provide additional information received from the ATX and send messages to the ATX to configure and monitor the functioning or apply the stimulation to the patient.

This system makes it possible to position the cables close to the patient, connected to an ATX, and place the corresponding RXA in the vicinity of the device being used.

Advantages of this system:
- Cables are shortened slightly and long cables are removed from the system (in the case of areas in which wireless connections are not possible, the number of cables would be reduced significantly and the use of fibre-optics is also an option).
- In principle, the same commercial devices used to date can still be used.
- The only system connected to the patient is the ATX, while the measuring, conversion and monitoring devices are not in physical contact with the patient. This makes it possible to simplify and reduce the cost of designs without the need for levels of protection and fail-safe features. They can also be moved away from the patient to increase the surgeon's work area.
- At a later stage, it will be possible to use the ATX directly connected to a data receiver and processing system, simply using a computer instead of the instruments.

The wireless electronic intraoperative monitoring system minimizes the wiring of systems used today to improve the field of action on the patient, while, of course, maintaining their features. This will let surgeons work with more room and with greater concentration on the operation, since they will not have to worry about separating the sensor wiring from their field of action or whether or not the sensors remain connected to the patient.

In short, the system consists of one or more transmitters with floating power using batteries or similar to replace analogue connection cables with internal and external electrodes with the following characteristics:
- Adapt to the measuring conditions autonomously.
- Provide an output that repeats the measured signal so that the instruments used at present continue to work correctly as if they were directly connected to the electrodes.
- On-demand evaluation of the contact resistance of the electrodes.
- Apply stimulation signals.
- Convert data for direct use on a computer system.
- It can be adapted to various dynamic range/band input and output/amplification conditions.

### Description of the figures

Figure 1 shows the monitoring system used at present.
Figure 2 shows the invention: the ATX/RXA system. This figure shows the minimization of the wiring and the presence of the ATX and RXA systems.

### Description of an embodiment

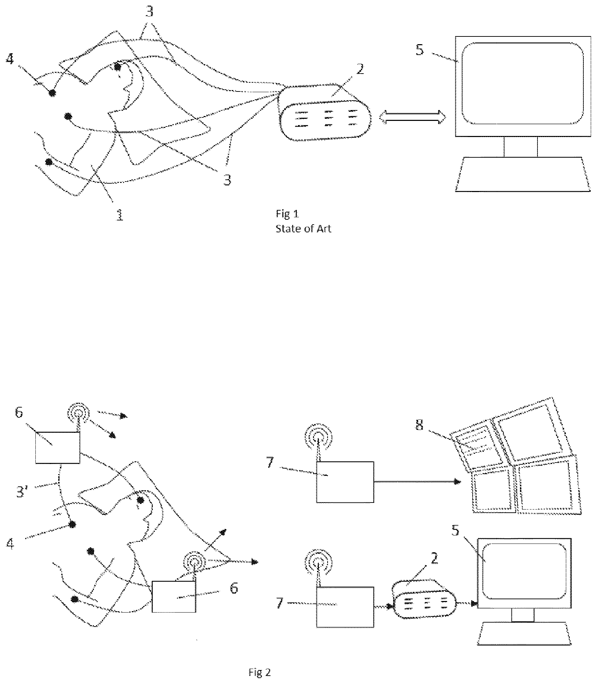

Figure 1 shows the state of the art, where a patient (1) is connected to a monitoring device (2) by means of cables (3) with sensors (4) or stimulators connected to the ends. The monitoring device is usually controlled from a computer (5) or similar.

The invention revises this system, the patient (1) being equally covered with sensors (4) or stimulators, which in this case can be increased, but in this case they are connected by a short cable (3') (generally shorter than 0.5 m) to one or more wireless conditioning-transmitting elements, ATX (6) and, in general, a receiver-actuator element, RXA (7), which receives the wireless signals and, where appropriate, forwards the response messages or stimulation commands to the ATX (6).

As shown in Figure 2, several RXA (7) can be used, e.g. one connected to the monitoring device (2) and the corresponding computer (5) and the second to a remote monitoring device (8).

One simple position of the ATX (6) is beneath the operating table or stretcher because it is out of sight and does not interfere with the surgeon's movements.

## Claims

1. Intraoperative monitoring system comprising a series of sensors (4) or stimulators placed on the body of a patient (1) and connected to one or more wireless signal-conditioning/-transmitting elements (ATX (6)) using small cables (3') and one or more wireless receiving/actuating elements (RXA (7)) connected to the monitoring system.

2. System, according to claim 1, wherein the ATX (6) are positioned beneath the operating table or stretcher.
